# EUROPEAN PATENT SPECIFICATION

(11) **EP 1 753 412 B1**
(45) Date of publication and mention of the grant of the patent: **18.01.2017**
(21) Application number: 05754185.6
(22) Date of filing: 09.06.2005
(51) Int. Cl.: A61K 9/70, A61K 9/00, A61K 31/00

(54) **SELF-HEATING PHARMACEUTICAL PATCH**
SELBSTERWÄRMENDES PHARMAZEUTISCHES PFLASTER
BANDE ADHESIVE PHARMACEUTIQUE AUTOCHAUFFANTE

(30) Priority: 09.06.2004 GB 0412793
(43) Date of publication of application: 21.02.2007
(73) Proprietor: Reckitt Benckiser Healthcare (UK) Limited, Slough, Berkshire SL1 3UH (GB)
(72) Inventor: PANKHANIA, Mahendra Govind, Bramcote, Nottingham NG9 3HP (GB); RAVEN, Stephen John, Willoughby on the Wolds Loughborough LE12 6TE (GB)
(74) Representative: O'Brien, Niall James
(86) International application number: PCT/GB2005/002251
(87) International publication number: WO 2005/120472

(56) References cited:
- EP-A- 0 218 759
- US-A- 4 963 360
- US-A- 5 658 583
- US-A- 6 099 556
- US-A1- 2002 004 066
- US-A1- 2002 119 186
- US-B1- 6 284 266
- US-B1- 6 465 709

## Description

This invention relates to a therapeutic product, and in particular to a therapeutic product which comprises a patch to be located on skin, to provide transdermal passage of a medicament.

Many such patches are currently available. One well known example is a nicotine patch used in the treatment of smoking addiction. Another example is an analgesic patch used to provide relief from back pain or menstrual cramp.

Such medicament patches are often seen as being a good clinical measure to adopt when the aim is to provide a supply of a medicament to a patient over an extended period.

A different type of patch does not deliver a medicament to a patient but rather, heat. Such patches may be of benefit in treating, for example, certain muscular conditions, for example arising from sports injuries. Heat may be obtained by promoting an exothermic reaction, for example a redox reaction between components retained within the patch, or a reaction between a component retained in the patch and an external agent, for example water or air.

There have been some proposals to provide a heated medicament patch. For example WO 93/07842 describes a series of different embodiments of heated medicament patches. One embodiment, that of Figure 2 of that patent application, has an internal heating means but this is a cumbersome overlay piece, on top of a basic medicament patch construction. The overlay piece has two chambers with a rupturable wall between them. The user must in some manner rupture the wall (without rupturing the external wall or damaging the patch) to obtain mixing of the first and second chemical species, which react and generate heat. This is an unpromising arrangement from the technical and commercial point of view. Other embodiments, shown in Figures 1 and 3 respectively of WO93/07842, require an external source of heat.

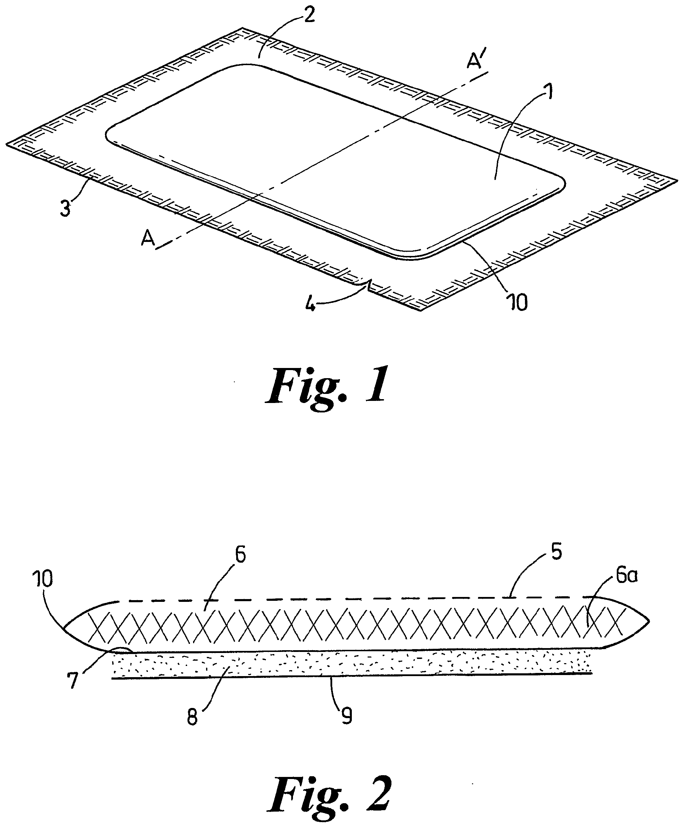

Another example of heated medicament patch is shown in US 4 879 119. Again, there is an embodiment of patch in which external heat is required (shown in Figure 1) and an embodiment of patch in which heat can be generated internally (Figure 2). The latter patch has a flat base portion for attachment to the skin and, standing up from the base portion, a deep overlay piece containing in one section a medicament and in another section the heating element, namely iron powder able to generate heat on oxidation. Again, the construction described for a self-heating medicament patch is not a practical one.

The attempts described above for providing a self-heating medicament patch are, it is submitted, technically unpromising and have not led, to our knowledge, to significant further published work, or to any commercial product.

Aside from the apparently impractical constructions shown in these patents such "patches" (if that is an appropriate word for such devices, with their bulky overlay pieces) would be unlikely to gain any consumer acceptance. They are likely also to be uncomfortable to wear and their outline is likely to show through clothing, unless it is very loose.

US 5 658 583 describes an apparatus, product formulation, and method for improved dermal permeation of pharmaceuticals wherein the apparatus includes a thin drug formulation reservoir and a heat-generating chamber separated by a first non-permeable wall, wherein the reservoir and chamber are formed in or supported by a housing. US 2002/0119186 is directed to a method and system for delivering a drug into the systemic circulation. US 6 284 266 describes methods and apparatus for improving administration of analgesics through the use of heat. US 2002/0004066 relates to a transdermal drug delivery system comprising a dermal drug delivery patch and a heating element compartment securable to the dermal drug delivery patch. US 4 963 360 discloses an exothermic package body having a carrier layer comprising a medicinal component, and an exothermic layer which develops heat when exposed to the air to enhance absorption of the medicinal component through the skin, hence improving medicinal effects. US 6 099 556 discloses an exothermic composition, enclosed in a container at least part of which is gas-permeable, generates heat in the presence of air. EP 0 218 759 describes a method of moxibustion which is the burning of moxa herb on the skin. In the method described a pyrogen is used to heat the moxa on contact with oxygen. US 6 465 709 relates to a multi-layer exothermic bandage comprising an oxygen-impermeable layer, a water impermeable layer, a heating element layer comprising an oxidizable material, and an active agent layer; and the use thereof.

It is an object of the present invention to provide a self-heating medicament patch which is of a construction which is more practical, more apt to gain user acceptance and more likely to have commercial value.

In accordance with the present invention there is provided a therapeutic product, being a patch which is of multilayer construction, and which produces heat when exposed to air, and an air-impermeable removable wrapping around the patch, wherein the patch comprises, in sequence:
an adhesive layer, by means of which the patch is, in use, secured to the skin;
a medicament layer, the medicament thereof being able to diffuse through the adhesive layer and into the skin;
or instead of a separate adhesive layer and medicament layer, a combined adhesive and medicament layer, the medicament thereof being able to diffuse from the combined adhesive and medicament layer and into the skin;
a carrier layer for the adhesive layer and the medicament layer, or for the combined adhesive and medicament layer;
a layer of a substance which undergoes an exothermic reaction upon exposure to air;
and an air-permeable backing layer;
wherein the patch has a completely sealed periphery, in the central region, inboard of the sealed periphery, the patch is substantially the same thickness throughout, is flat and flexible, and is of thickness not exceeding 6mm.

Described above is preferably the order of components of the patch when the patch is in use: the medicament and/or adhesive layer is in contact with the skin and the backing layer is the outermost exposed layer.

The product of the present invention offers several important health benefits, including: the application of heat to a body site which can benefit therefrom; the simultaneous delivery of medicament directly to that site; and the acceleration of that delivery, by the action of the heat. It may be expected that the diffusion and absorption of the medicament may be promoted, both through the patch and through the body of the person being treated.

The substance which undergoes an exothermic reaction upon exposure to air is present so as to provide the patch with an internal heat source. This may be described as a heat generating or heat-producing substance, or heating means.

An important distinction between a therapeutic patch of the present invention and a self-heating therapeutic patch of the prior art described above is that the former is of generally flat form. The thickness of a patch of the present invention does not exceed 6 mm, preferably at any location. Preferably its thickness does not exceed 5 mm at any location. More preferably its thickness does not exceed 3 mm at any location. Unlike patches of the prior art it does not have a part upstanding from the plane of the patch, to accommodate a heating means. It may be laid flat, on a flat surface, such as a table top. It may be supplied flat. However, because it is flexible it may suitably be moved into different positions out of its plane. Thus, it may adopt the shape of a body part to which it is applied. If that shape changes as the patient moves, the patch is preferably sufficiently flexible to accommodate the changes, and the patch can be held securely on the skin.

The thickness of the central region may suitably be slightly greater than that of the sealed periphery, because of the compression thereof on sealing and, because the substance which undergoes an exothermic reaction upon exposure to air is distributed throughout this central region. Preferably, however, a patch of the present invention does not have any substantial discontinuities in its thickness.

Thus, the patch used in the present invention could be similar in construction to a conventional therapeutic patch without any heating means, as it is of suitably slim form. When adhered to skin it is suitably unobtrusive. Preferably it does not have an outline which would show through clothing, even tight, elasticated, clothing.

Preferably the patch is of the passive type. That is there is preferably no electronic means for promoting or adjusting the diffusion rate of the medicament. Preferably the patch has no electrical power source.

Preferably the substance which undergoes an exothermic reaction upon exposure to air comprises iron, preferably in the form of iron powder or iron filings. Alternatively, a mixture of an alkaline sulfide and iron carbide could be used. In addition to such an oxidisable material, materials may be present to modify - accelerate or decelerate - the oxidation. These materials may include mineral salts (e.g. NaCl), water, sand, wood dust, charcoal and vermiculite. Preferably, materials which reduce the rate of release of heat are present, so that a moderately elevated temperature - within the limits stated below - is maintained for an extended period.

Preferably the heat-producing substance is such that, in use, a thermocouple located (for the purpose of this test) on the side of the patch which makes contact with the skin in use shows a temperature of at least 38°C, more preferably at least 42°C. Preferably, such a thermocouple shows a temperature not in excess of 50°C, more preferably not in excess of 45°C. Preferably the thermocouple denotes a temperature within the ranges stated above for a continuous period of at least 2 hours, more preferably at least 4 hours, and most preferably at least 6 hours.

The patch is secured to the skin by means of an adhesive composition which is carried on the patch by the carrier layer, preferably as manufactured. The adhesive may be a hydrogel adhesive, but could also be an adhesive of non-hydrogel type.

In embodiments in which the medicament layer is provided separately to the adhesive layer, the adhesive is preferably permeable to the medicament. Examples of adhesives are polysiloxanes, polyacrylates, plasticised ethylene-vinyl acetate copolymers, polyurethanes, and elastomeric polymers such as polyisobutene.

Preferably any adhesive layer is covered in manufacture by a release layer.

In certain embodiments of the present invention the medicament may be compounded with the adhesive. A patch of such an embodiment may therefore comprise an air-permeable backing layer, a heat-generating substance, a carrier layer, an adhesive/medicament layer, and a release layer.

In certain other embodiments the patch could comprise an air-permeable backing layer, a heat-generating layer, a carrier layer, a layer comprising the medicament, an adhesive layer, and a release layer.

In certain embodiments in which there is a combined adhesive and medicament layer, the patch could comprise an air-permeable backing layer, a heat-generating layer, a carrier layer, and a layer comprising one or more adhesive zones and one or more medicament zones, separate from said one or more adhesive zones. For example there may be a medicament zone with an adhesive zone around it.

A release layer mentioned above is a disposable element which serves to protect the patch prior to application. Preferably the release layer is of a material covering the adhesive, impermeable to the medicament and adhesive, and easily stripped from the adhesive. Suitable release layers include papers or polymers treated with silicone or fluorocarbon agents. Silicone-coated polyester sheets are especially preferred.

The air-permeable backing layer and the carrier layer together provide an enclosure in which the heat-producing substance is contained. The carrier layer also provides a surface on which the adhesive and medicament layer(s) are carried. The backing layer must be air-permeable to permit air to reach the heat-producing substance contained therein but the carrier layer is preferably impermeable to the adhesive and medicament to prevent migration in the opposite direction to that which is desired.

The heat-producing substance may be retained in the patch in the form of an envelope or pouch or sachet formed by completely sealing around their periphery the air-permeable backing layer and the carrier layer. The backing layer and carrier layer may together form a backing or substrate which surrounds the heat-producing substance. Alternatively, the backing or substrate may be formed from a single sheet which is folded and then sealed along its three open edges, around the heat-producing substance, so that the heat-producing substance cannot escape from the patch i.e. it is sealed about the periphery.

Preferably the carrier layer carries the medicament and adhesive layer(s) on its outside and is impermeable to them. Alternatively, if the carrier backing layer was formed from a material permeable to the medicament and adhesive, an additional plastics sheet could be supplied within the patch or on its outside, impermeable to the medicament and adhesive.

The backing layer may comprise a material which is itself air-permeable, for example a fabric; or it may comprise a material which is itself impermeable to air but which is provided with slits or other perforations to permit air to permeate through it and reach the heat-producing substance, i.e. it is air-transmissive. Alternatively, the backing layer may be provided with windows of air-permeable material.

The backing layer and the carrier layer may be made from the same material, which is preferably permeable to air, non-transmissive of the heat-producing substance, impermeable to the medicament and impermeable to the adhesive. Suitable materials may include plastics or elastomeric materials and proofed paper and fabric products.

Examples of suitable elastomeric polymers may include polyether amide block copolymers, polyethylene methyl methacrylate block copolymers, polyurethanes, silicone elastomers, rubber-based polyisobutylene, styrene, and styrene-butadiene and styrene-isoprene copolymers. Examples of suitable flexible plastics materials include polyethylene, polypropylene, and polyester.

Preferably the heat-producing substance is held loosely between the carrier layer and the backing layer. By held loosely is meant that the movement of the material is not restricted. For example, when the heat-producing substance comprises iron filings, these are free to move in the region between the backing layer and the carrier layer and are not immobilised in any way, within that region.

The medicament to be released by the patch may be selected, typically, from analgesics, anti-inflammatory agents, corticosteroids, chemotherapeutic agents, anaesthetics, antihistamines, anti-infectives, antibacterials, anti-parasitics, anti-viral agents, anti-oxidants, immunomodulators ceratolytics, and antineoplastics. This invention is of particular interest in relation to the delivery of analgesics and anti-inflammatory agents, especially non-steroidal anti-inflammatory agents (NSAIDS). Preferred NSAIDS may be selected from NSAIDS have melting points in the range 30-200°C (such as racemic naproxen, melting point 156°C), more preferably 30-150°C, further preferably 40-120°C (such as racemic flurbiprofen, melting point 114°C), most preferably 40-100°C (such as racemic ibuprofen (melting point 75-77°C), S(+)-ibuprofen (melting point 52-54°C) and racemic ketoprofen (melting point 96°C)). Preferred low-melting NSAIDs are naproxen, ketoprofen, flurbiprofen, and ibuprofen. Preferably the NSAID is in the form of a racemic mixture or an enantiomer (especially the S(+)-enantiomers) thereof. Salts of racemic NSAIDS and enantiomers thereof may also be used. Preferred salts are the sodium salts, potassium salts and organic salts, for example salts of amino acids, such as arginine and lysine.

Particularly preferred medicaments for use in the present invention are naproxen, meloxicam, flurbiprofen, diclofenac, ketoprofen and, especially, ibuprofen.

The invention is especially adapted for an ibuprofen medicament. The term "ibuprofen medicament" preferably includes racemic ibuprofen and S(+)-ibuprofen and their sodium, potassium and lysine salts which have low melting points and a very poor after-taste in the mouth and throat. Most advantageous results are obtained with racemic ibuprofen which has a high dosage combined with poor solubility properties.

The medicament may, if desired, include other compatible pharmacologically active ingredients and/or enhancing agents. Thus, for example, the dosage form may include any other ingredient commonly used in a composition useful to treat pain, inflammation and/or fever, for example caffeine or another xanthine derivative, another analgesic, for example codeine, a skeletal muscle relaxant: an antihistamine (e.g. acrivastine, astemizole, azatadine, azelastine, bromodiphenhydramine, brompheniramine, carbinoxamine, cetirizine, chlorpheniramine, cyproheptadine, dexbromopheniramine, dexchloropheniramine, diphenhydramine, ebastine, ketotifen, lodoxamide, loratidine, levocabastine, mequitazine, oxatomide, phenindamine, phenyltoloxamine, pyrilamine, setastine, tazifylline, temelastine, terfenidine, tripelennamine or triprolidine (preferably non-sedating antihistamines are employed)); a decongestant (e.g. pseudoephedrine, phenylpropanolamine and phenylephrine); a cough suppressant (e.g. caramiphen, codeine or dextromethorpan); and/or an expectorant (e.g. guaifenesin, potassium citrate, potassium guaiacolsuphonate, potassium sulphate and terpin hydrate).

The patch is supplied in an air-impermeable wrapping, which may enclose the patch in a vacuum, or in an inert gas, such as nitrogen. However it may be acceptable to leave air within the wrapping. The effect of this small amount of air on the viability of the heating means is likely to be negligible.

Such wrapping can be any sheet material of low air-permeability. It may be a metallic foil, or a plastics material, or a plastics-foil laminate. Persons skilled in the art will be well aware of suitable materials and how they should be sealed (preferably two sheets embracing the patch, the two sheets being hermetically sealed together around their entire periphery). No more needs to be said here, to assist the skilled person.

The patch can be of any shape, for example rectangular, square, circular, oval etc. Preferably its area is at least 20 cm², more preferably at least 50 cm². Preferably its area does not exceed 400 cm², more preferably does not exceed 300 cm², and most preferably does not exceed 200 cm³.

The invention will now be further described, by way of example, with reference to the accompanying drawings in which:
Fig. 1 is a perspective view of a standard size patch in accordance with the invention, retained within an airtight pocket;
Fig. 2 is a cross-section of the patch shown in Fig. 1, along line A-A'.
Fig. 3 shows a perspective view of the upper side of a second embodiment of patch, with its cover in place;
Fig. 4 shows a perspective view of the upper side of the patch of Fig. 3 with the cover pulled back;
Fig. 5 is a longitudinal cross section of the patch of Figs. 3 and 4, along the line B-B';
Fig. 6 is a perspective view of an alternative patch, formed from two patch elements which are shown in Figs. 7 and 8; and
Figs. 9 to 11 are cross-sectional views of the articles shown in Figs. 6 to 8, along the lines C-C' shown in the latter drawings.

With reference firstly to Figs. 1 and 2, a rectangular patch 1 is sealed in an airtight pocket 2 which is filled with nitrogen. The pocket 2 is formed by two sheets of metallic/plastics laminated foil, which are hermetically sealed at the periphery of each. These form an endless seal 3. The dimensions of the pocket are slightly larger than the patch to allow the patch to fit inside easily. There is a small cut 4 which extends across most of the seal 3. This facilitates opening the foil pocket by tearing across one end.

The upper surface of the patch 1 comprises an upper semi-permeable backing layer or sheet 5, which allows air to enter region 6. The lower surface of the patch (the carrier layer) comprises an impermeable membrane, in this embodiment a polyester sheet 7. Sheets 5 and 7 are heat sealed around their common periphery 10 to provide region 6 which houses a heat-generating composition 6a comprising iron filings, as an oxidisable material. A mixture 8 of adhesive and medicament coats the lower surface of sheet 7, sheet 7 being impermeable to mixture 8. The mixture 8 comprises a mixture of an adhesive containing sodium polyacrylate, and also contains ibuprofen. The adhesive permits diffusion of the ibuprofen, leading to its transdermal absorption. The adhesive/medicament layer is covered by a release layer 9 which comprises a silicone-coated polyester sheet and forms the lower surface of the patch. The release layer is impermeable to the adhesive/medicament.

In use, patch 1 is removed from pocket 2 immediately prior to application. Upon this exposure to air, oxygen can penetrate semi-permeable membrane 5 thus initiating exothermic oxidation of the iron filings within the heat-generating composition 6a. Release layer 9 is removed to uncover the adhesive, and the patch applied to the skin. The patch is flexible and may be applied, if wished, to curved parts of the body.

The patch of Figs. 1 and 2 is a large patch (approximately 20 cm long, 15 cm wide and 3 mm thick in this embodiment) and could, for example, be applied to the lower back in the treatment of back ache.

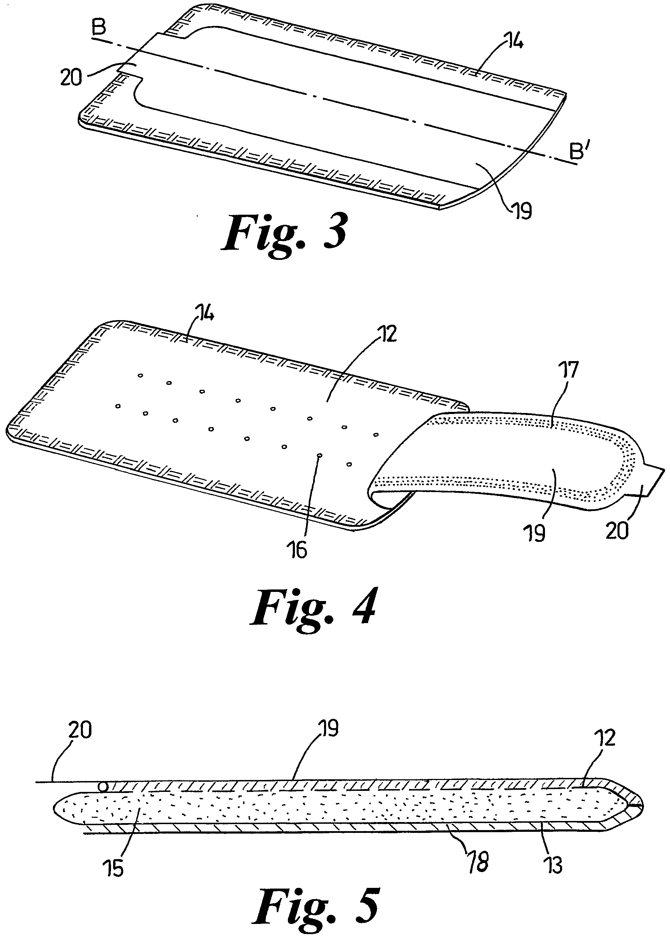

By way of example, the patch 11 of Figs. 3 to 5 is smaller (approximately 10 cm long, 4 cm wide and 3 mm thick), and is suitable for application to the forehead to treat a headache.

Patch 11 comprises two polyester sheets, 12 and 13 (as the backing layer and the carrier layer), which are secured together by heat sealing all around their common periphery 14 (alternatively one edge may be folded). There is formed therebetween a cavity which houses a heat-releasing composition 15, containing iron filings. Upper polyester sheet 12 (the backing layer) bears a plurality of microscopic perforations 16 which render the exposed sheet permeable to the air. Around the edge of the perforated section, there is a bead 17 of a rubber-like adhesive. On the bottom surface of lower polyester sheet 13 (the carrier layer) there is a layer 18 comprising a mixture of an adhesive containing sodium polyacrylate, and ibuprofen. A single silicone-coated polyester sheet 19 bends to cover both the top of upper polyester sheet 12 and the bottom of lower polyester sheet 13. The bead 17 of rubber-like adhesive is chosen so that an airtight seal is formed between the top of the upper polyester sheet 12 and the top section of cover 19. It is especially preferred that the bead of the rubber-like adhesive 17 is removed with the cover i.e. that it binds more strongly to cover 13 than it does to polyester sheet 16. On the other hand adhesive found in mixture 18 is not removed with cover sheet 19. Rather, it remains upon the bottom of polyester sheet 13. The ibuprofen compounded into mixture 18 remains bound to the lower surface of the patch. The removal of covering layer 19, which is impermeable to ibuprofen, is facilitated by the inclusion of a tab 20.

Patch 11 is also flexible, and can be applied, and will firmly adhere to, curved parts of the body.

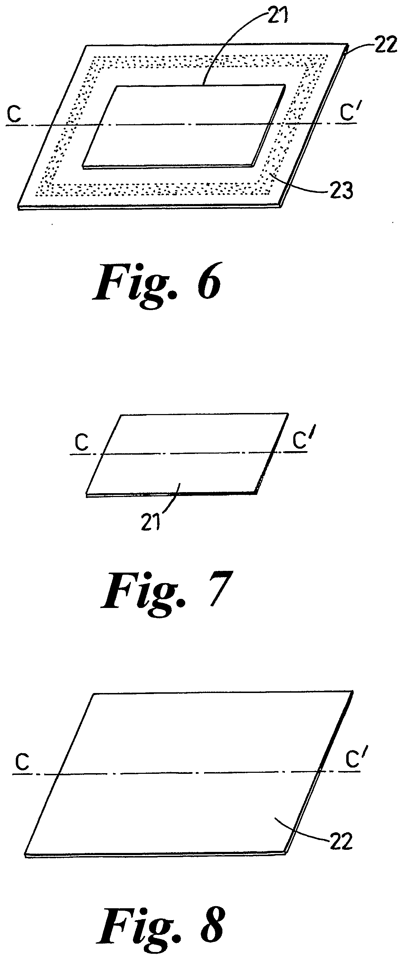

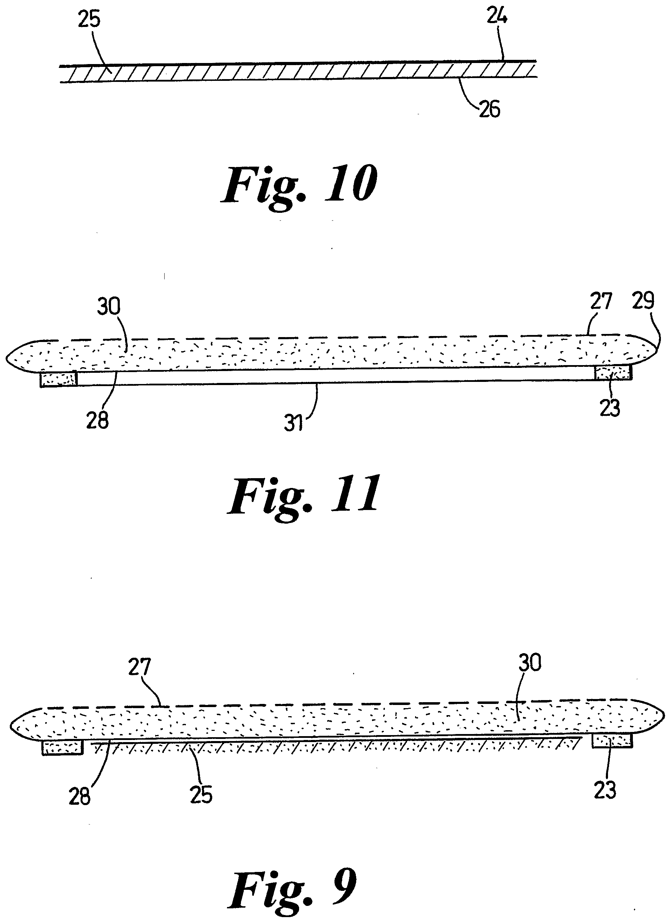

A third embodiment shown in Figs. 6 and 9 consists of two parts. Figs. 7 and 10 show a medicament patch 21 which is similar in construction to a conventional sticking plaster. The substantive difference is that it carries a medicament. Figs. 8 and 11 show a separate heating patch 22, which is larger than medicament patch 21. Heating patch 22 has adhesive 23 around the periphery so that it can be applied on top of the medicament patch 21, as shown best in Fig. 9.

Medicament patch 21 consists essentially of a fabric layer 24 of the type used to make traditional sticking plasters, the lower surface of which is coated by a mixture 25 containing adhesive and medicament. There is a silicone-coated polyester cover layer 26 which is removed prior to application. The medicament patch is of thin and flexible form.

Heat patch 22 is supplied in a hermetically-sealed envelope which is not shown. It comprises two polyester sheets, 27 and 28, which are heat-sealed entirely around their periphery 29. The cavity created therebetween houses heating composition 30, which contains iron filings. Upper sheet 27 (the backing layer) contains microscopic pores. Thus when the patch is removed from the envelope, the composition 30 is exposed to air and heating is induced. The periphery of lower sheet 28 carries a layer of adhesive 23 which is covered by a silicone coated polyester sheet 31.

Thus, in use, cover 26 is removed from medicament patch 21 which is applied to the skin. Heat patch 22 is then removed from the lower surface to reveal adhesive 23 and the heat patch is placed over the medicament patch as shown in Fig. 9.

Patches of various other shapes and sizes having features in common with the previous examples are also envisaged. The possibility further exists for a pack comprising a number of patches of differing size.

## Claims

1. A therapeutic product, being a patch which is of multilayer construction, and which produces heat when exposed to air, and an air-impermeable removable wrapping around the patch, wherein the patch comprises, in sequence:
an adhesive layer, by means of which the patch is, in use, secured to the skin;
a medicament layer, the medicament thereof being able to diffuse through the adhesive layer and into the skin;
or instead of a separate adhesive layer and medicament layer, a combined adhesive and medicament layer, the medicament thereof being able to diffuse from the combined adhesive and medicament layer and into the skin;
a carrier layer for the adhesive layer and the medicament layer, or for the combined adhesive and medicament layer;
a layer of a substance which undergoes an exothermic reaction upon exposure to air;
and an air-permeable backing layer;
wherein the patch has a completely sealed periphery, in the central region, inboard of the sealed periphery, the patch is substantially the same thickness throughout, is flat and flexible, and is of thickness not exceeding 6mm.

2. A therapeutic product according to claim 1 wherein the layer of substance which produces heat when exposed to air comprises iron powder or iron filings.

3. A therapeutic product according to claim 1 or 2 wherein the layer of substance which produces heat when exposed to air further comprises a moderator substance to reduce the rate of heat output from the patch.

4. A therapeutic product according to any preceding claim where the medicament is selected from: analgesics, anti-inflammatory agents, corticosteroids, chemotherapeutic agents, anaesthetics, antihistamines, anti-infectives, anti-bacterials, anti-parasitics, anti-viral agents, anti-oxidants, immunomodulators ceratolytics, and anti-neoplastics.

5. A therapeutic product according to any preceding claim where the medicament is a non-steroidal anti-inflammatory agent.

## Patentansprüche

1. Therapeutisches Produkt, das ein Pflaster ist, das einen mehrschichtigen Aufbau aufweist und Wärme erzeugt, wenn es gegenüber Luft exponiert wird, und eine luftundurchlässige, entfernbare Umhüllung um das Pflaster aufweist, wobei das Pflaster der Reihe nach umfasst:
eine Haftschicht, mit deren Hilfe das Pflaster in Verwendung an der Haut befestigt wird;
eine Arzneimittelschicht, deren Arzneimittel fähig ist, durch die Haftschicht und in die Haut zu diffundieren;
oder anstelle einer getrennten Haftschicht und Arzneimittelschicht eine kombinierte Haft- und Arzneimittelschicht, deren Arzneimittel fähig ist, aus der kombinierten Haft- und Arzneimittelschicht in die Haut zu diffundieren;
eine Trägerschicht für die Haftschicht und die Arzneimittelschicht oder für die kombinierte Haft- und Arzneimittelschicht;
eine Schicht aus einem Stoff, der bei Exposition gegenüber Luft eine exotherme Reaktion durchläuft;
eine luftdurchlässige Rückschicht;
wobei das Pflaster einen vollständig abgedichteten Außenumfang aufweist, wobei das Pflaster in dem zentralen Bereich innerhalb des abgedichteten Außenumfangs durchgehend eine im Wesentlichen gleiche Dicke aufweist, flach und flexibel ist und eine Dicke von nicht mehr als 6 mm aufweist.

2. Therapeutisches Produkt gemäß Anspruch 1, wobei die Schicht des Stoffs, der bei Exposition gegenüber Luft Wärme erzeugt, ein Eisenpulver oder Eisenspäne umfasst.

3. Therapeutisches Produkt gemäß Anspruch 1 oder 2, wobei die Schicht des Stoffs, der bei Exposition gegenüber Luft Wärme erzeugt, ferner einen Moderatorstoff zum Verringern der Wärmeabgaberate aus dem Pflaster umfasst.

4. Therapeutisches Produkt gemäß einem vorstehenden Anspruch, wobei das Arzneimittel ausgewählt ist aus: Analgetika, entzündungshemmenden Mitteln, Kortikosteroiden, Chemotherapeutika, Anästhetika, Antihistaminika, Antiinfektiva, antibakteriellen Mitteln, Antiparasitenmitteln, antiviralen Mitteln, Antioxidationsmitteln, Immunmodulatoren, Keratolytika und antineoplastischen Mitteln.

5. Therapeutisches Produkt gemäß einem vorstehenden Anspruch, wobei das Arzneimittel ein nichtsteroides entzündungshemmendes Mittel ist.

## Revendications

1. Produit thérapeutique, qui est un timbre de structure multicouche, et qui produit de la chaleur en cas d'exposition à l'air, et emballage amovible imperméable à l'air autour du timbre, où le timbre comprend, les unes à la suite des autres :
une couche adhésive, par le biais de laquelle le timbre est fixé à la peau pendant son utilisation ;
une couche de médicament, dont le médicament est capable de se diffuser à travers la couche adhésive et dans la peau ;
ou au lieu d'une couche adhésive et d'une couche de médicament distinctes, une couche adhésive et de médicament combinée, dont le médicament est capable de se diffuser à partir de la couche adhésive et de médicament combinée et dans la peau ;
une couche de support pour la couche adhésive et la couche de médicament, ou pour la couche adhésive et de médicament combinée ;
une couche d'une substance subissant une réaction exothermique en cas d'exposition à l'air ;
et une couche de support perméable à l'air ;
où la périphérie du timbre est entièrement scellée, et dans la région centrale, à l'intérieur de la périphérie scellée, le timbre présente substantiellement la même épaisseur partout, est plat et flexible, et son épaisseur ne dépasse pas 6 mm.

2. Produit thérapeutique selon la revendication 1, où la couche de substance qui produit de la chaleur en cas d'exposition à l'air comprend de la poudre de fer ou de la limaille de fer.

3. Produit thérapeutique selon la revendication 1 ou 2, où la couche de substance qui produit de la chaleur en cas d'exposition à l'air comprend en outre une substance modératrice réduisant la vitesse de dégagement de chaleur depuis le timbre.

4. Produit thérapeutique selon l'une quelconque des revendications précédentes, où le médicament est choisi parmi les suivants : analgésiques, agents anti-inflammatoires, corticoïdes, agents chimiothérapiques, anesthésiques, antihistaminiques, agents anti-infectieux, agents antibactériens, agents antiparasitaires, agents antiviraux, agents antioxydants, immunomodulateurs, kératolytiques, et antinéoplasiques.

5. Produit thérapeutique selon l'une quelconque des revendications précédentes, où le médicament est un anti-inflammatoire non stéroïdien.
